# EUROPEAN PATENT APPLICATION

(11) **EP 3 933 403 A1**
(43) Date of publication of application: **05.01.2022**
(21) Application number: 20182767.2
(22) Date of filing: 29.06.2020
(51) Int. Cl.: G01N 33/22, G01N 21/35, G01M 3/38

(54) **NATURAL GAS AND HYDROGEN DETECTOR AND METHOD FOR DETECTING THE CONCENTRATION OF HYDROGEN WITHIN A GAS SAMPLE**

(71) Applicant: Inficon GmbH, 50968 Köln (DE)
(72) Inventor: ENQUIST, Fredrik, Westmansgatan 49 (SE)
(74) Representative: dompatent von Kreisler Selting Werner - Partnerschaft von Patent- und Rechtsanwälten mbB

(57) **Abstract**

A natural gas and hydrogen detector (10) comprises at least one non-dispersive infrared (NDIR) sensor (12, 14) which is selectively sensitive to hydrocarbon gas components, at least one flammable gas sensor (16, 18) which is selectively or non-selectively sensitive to hydrogen gas, and an evaluation device (20) connected to the NDIR sensor and to the flammable gas sensor for receiving measurement values from the NDIR sensor and from the flammable gas sensor, the evaluation device being adapted to evaluate the measurement values by assessing whether an amount of hydrocarbon gas has been detected and whether an amount of a flammable gas component has been detected within the gas sample, and being adapted to consider hydrogen to be present within the gas sample if the amount or concentration of hydrocarbon gas detected is lower than the total amount or concentration of flammable gas components detected within the gas sample.

## Description

The invention relates to detection of hydrogen gas within a gas sample comprising natural gas.

An increasing need exists for detection of hydrogen within a gas sample comprising natural gas. For example, hydrogen is transported within natural gas pipelines where the natural gas is mixed with up to 20 % hydrogen, because an amount of up to 20 % hydrogen does not require any changes to appliances combusting the natural gas. Common conventional natural gas pipelines transport natural gas from the source of origin to the final consumer over long distances. Natural gas may typically consist of methane or mainly comprise methane in combination with further gas components. A common alternative to natural gas consisting of mainly methane is liquefied petroleum gas (LPG) consisting of propane or butane or a mixture of the two. Any reference to natural gas below indicates methane, a gas mixture mainly comprising methane, LPG, or a gas mixture comprising a combination of methane, ethane, propane and/or butane.

Natural gas and hydrogen are highly flammable. For safety reasons, it is important to be able to detect small leaks in the natural gas pipelines carrying natural gas and hydrogen. The leak detection is to be carried out during regular leak surveys and during commissioning testing. Moreover, it is desired that the actual ratio of hydrogen within natural gas can be measured.

The natural gas and hydrogen detector of the invention is defined by the features of claim 1. The method of the invention is defined by the features of independent claim 9.

The natural gas and hydrogen detector of the invention comprises at least one non-dispersive infrared (NDIR) sensor which is selectively sensitive to hydrocarbon gas components, as well as at least one hydrogen sensor which is selectively or non-selectively sensitive to hydrogen gas. An NDIR sensor, such as described in EP 1193488 A1, is an optical sensor with a sample chamber for the gas sample to be analysed, as well as an optical emitter and an optical receiver, for example on opposing sides of the sample chamber, such that infrared radiation emitted by the optical emitter travels through the gas within the sample chamber before reaching the optical receiver. Optical wavelengths absorbed by gas component within the gas sample are detected, such that wavelength peaks in the received infrared spectrum permit to identify gas components within the analysed sample. NDIR sensors can be optimized for detecting any IR active gas by selecting the appropriate infrared wavelength or wavelength range.

Hydrocarbon sensitive NDIR sensors typically operate at around 3.3 µm. The hydrogen molecule, however, is not infrared active and a hydrocarbon NDIR sensor is therefore not sensitive to hydrogen. In the following, "selectively sensitive" means that a specific gas component or group of components can be identified by the sensor, whereas "non-selective" means that a sensor is able to detect the presence of a gas component, but is not able to identify the specific gas component.

Accordingly, the flammable gas sensor of the natural gas and hydrogen detector of the invention is either selectively or non-selectively sensitive to hydrogen gas. In the case of a hydrogen selective flammable gas sensor, the sensor is able to identify that the detected gas component is hydrogen. In the case of a non-selective flammable gas sensor, the sensor is only able to detect that flammable gas components are present within the sample, such as hydrogen or other flammable gas components, but without being able to identify hydrogen or the amount of hydrogen within the sample.

Combining such flammable gas sensor with a hydrocarbon NDIR sensor permits to identify that hydrogen is present, if the flammable gas sensor reacts to the gas sample, while the NDIR sensor does not. Hydrogen is one of few naturally occurring flammable gases that are not infrared active, i. e., which cannot be detected with an NDIR sensor. All flammable gases occurring in noticeable concentrations in natural gas are IR active and absorbing noticeably in the commonly used 3.3 µm range. This means that if a non-selective flammable gas sensor reacts to a gas sample, while a hydrocarbon specific NDIR sensor does not react to the sample, this is a very strong indication of hydrogen being present within the sample. This combo of sensors permits a super selective hydrogen analysis, as well as the possibility to detect and quantify the hydrogen content within a natural gas sample in the range of parts per million (ppm) or even lower and up to 100 volume percent (vol%) of hydrogen within the sample.

The natural gas and hydrogen detector of the invention comprises an evaluation device connected to the NDIR sensor and to the flammable gas sensor in order to receive the measurement values from both sensors. The evaluation device is adapted to evaluate the measurement values by assessing whether an amount of hydrocarbon gas has been detected and whether an amount of a flammable gas component has been detected within the gas sample.

The evaluation device considers hydrogen to be present within the gas sample, if the amount or concentration of hydrocarbon gas detected is lower than the total amount or concentration of flammable gas components detected within the sample. In particular, the hydrocarbon gas amount/concentration within the sample is determined from the NDIR sensor measurement values, and the flammable gas amount/concentration within the sample is determined from the flammable gas sensor measurement values, whereafter the hydrocarbon gas amount/concentration may be subtracted from the flammable gas amount/concentration.

If the result of the subtraction is below a certain threshold or zero, no hydrogen is present within the sample. On the other hand, if the result of the subtraction is above a certain threshold, hydrogen gas is considered to be present within the gas sample.

The flammable gas sensor may be non-selective to hydrogen, but sensitive to flammable gases, and may be combined with a hydrocarbon NDIR sensor for selective detection and quantification of low concentrations of hydrogen within the natural gas sample up to a few volume percent.

Alternatively or in addition, a thermal conductivity (TC) sensor, a speed of sound sensor and/or a high range non-selective flammable gas sensor, may be employed as a flammable gas sensor in combination with a hydrocarbon NDIR sensor for higher concentrations from typically fractions of a percent up to 100 vol% of hydrogen within natural gas. A speed of sound sensor measures the speed of sound in the gas and reacts primarily to hydrogen in this context. It is also very sensitive to helium and also, but less, sensitive to methane. All in all, the characteristics of a speed of sound sensor are similar to that of a TC sensor.

On the other hand, an INFICON FET sensor, such as described in AU 508461 A or in GB 1520456 A may be employed as a selective flammable gas sensor, thus showing no significant response to methane, ethane, propane or butane, in combination with a hydrocarbon NDIR sensor. As a result, there is no overlap in non-hydrocarbon compound sensitivity of the gases detected by the two sensors, thereby increasing the selectivity of the natural gas and hydrogen detector of the invention. Thus, a combination of a hydrogen selective flammable gas sensor, such as the INFICON FET sensor, with a hydrocarbon NDIR sensor is suitable for hydrogen contents within the sample of below 1 ppm up to a few thousand ppm of hydrogen within a natural gas plus hydrogen fuel gas mixture.

In particular, the invention permits to employ two different flammable gas sensors in combination with a hydrocarbon NDIR sensor. The flammable gas sensors may be a thermal conductivity sensor, a speed of sound sensor and/or a high range non-selective flammable gas sensor, and a non-selective but sensitive flammable gas sensor or a hydrogen selective flammable gas sensor. The flammable gas sensor can, for example, be a general flammable gas sensor such as a metal oxide sensor, often referred to as a Taguchi or SnO₂ sensor, a pellistor sensor, a TC sensor, a speed of sound sensor or a selective flammable gas sensor, such as a FET sensor. The Taguchi sensor is typically used from ppm or sub-ppm range to several or a few percent of hydrogen within the sample. A pellistor is suitable for fractions of a percent up to a few percent of hydrogen content. A TC sensor can typically be used from hundreds of ppm up to 100 vol% and, in this context, reacts primarily to hydrogen and methane.

In general, the output signal of the hydrocarbon NDIR sensor is treated by algorithms for linearization and calibration of the measurement signal in order to generate the total hydrocarbon gas concentration. This value is subtracted from a similarly treated output signal of the non-selective general flammable gas sensor, non-selective TC sensor or speed of sound sensor. Thereby, the respective concentration of hydrocarbons and hydrogen is generated.

The NDIR sensor may be sensitive to a first range of hydrocarbon components, while a second NDIR sensor is employed for detection of a second range of hydrocarbon concentrations being different form the first range. Alternatively, a multi-wavelength NDIR sensor may be employed, in which multiple optical filters with different pass bands for IR transmission are employed. Behind every optical filter of such multi-wavelength NDIR sensor, a separate optical receiver is located.

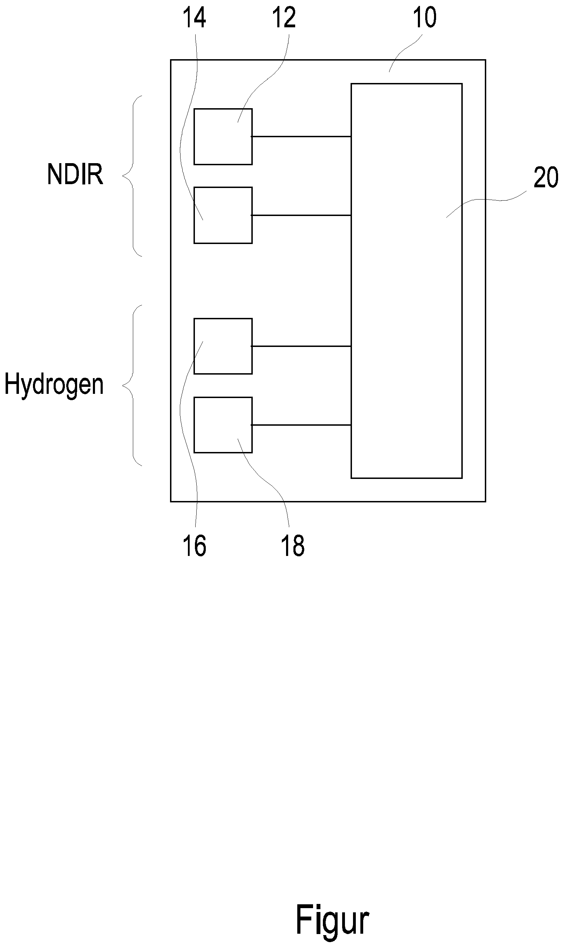

In the following, an example of the invention is explained with regard to the figure, in which an embodiment of the invention is shown.

The figure shows an embodiment of the natural gas and hydrogen detector 10 comprising two NDIR sensors 12, 14 and two flammable gas sensors 16, 18. All sensors 12, 14, 16, 18 are connected to an evaluation device 20, such that the measurement values from all sensors 12, 14, 16, 18 are transmitted in the form of electronic data to the evaluation device 20. The evaluation device 20 comprises a microprocessor or computer which is adapted to assess whether an amount of hydrocarbon gas has been detected by the NDIR sensors, and whether a presence of a flammable gas component has been detected within the gas sample by one of the flammable gas sensors. The evaluation device 20 considers hydrogen to be present within the gas sample, if the amount of hydrocarbon gas detected is lower than the total amount of flammable gas components detected within the sample. In particular the hydrocarbon gas concentration within the sample is determined from the NDIR sensor measurement values, and the flammable gas concentration within the sample is determined from the flammable gas sensor measurement values, whereafter the hydrocarbon gas concentration is subtracted from the flammable gas concentration.

The first NDIR sensor 12 has a short infrared sample path within the measurement sample chamber, and is thereby used for high concentrations, while the second NDIR sensor 14 has a long sample path within the measurement sample chamber used for low hydrocarbon concentrations. Alternatively or in addition, a multi-channel infrared sensor may be used as a NDIR sensor with one optical filter, typically centred around 3,3 µm, for low concentrations, and another filter is used for wavelengths with lower hydrocarbon absorption to detect the higher concentrations.

The first flammable gas sensor 16 can be a general flammable gas sensor or a hydrogen selective gas sensor, while the second flammable gas sensor 18 can, for example, be a thermal conductivity sensor or a speed of sound sensor.

## Claims

1. Natural gas and hydrogen detector (10) comprising
at least one non-dispersive infrared (NDIR) sensor (12, 14) which is selectively sensitive to hydrocarbon gas components,
at least one flammable gas sensor (16, 18) which is selectively or non-selectively sensitive to hydrogen gas, and
an evaluation device (20) connected to the NDIR sensor and to the flammable gas sensor for receiving measurement values from the NDIR sensor and from the flammable gas sensor, the evaluation device being adapted to evaluate the measurement values by assessing whether an amount of hydrocarbon gas has been detected and whether an amount of a flammable gas component has been detected within the gas sample, and being adapted to consider hydrogen to be present within the gas sample if the amount or concentration of hydrocarbon gas detected is lower than the total amount or concentration of flammable gas components detected within the gas sample.

2. Natural gas and hydrogen detector according to claim 1, wherein the NDIR sensor is not sensitive to hydrogen and/or wherein the flammable gas sensor is a hydrogen selective sensor.

3. Natural gas and hydrogen detector according to claim 1 or 2, wherein the flammable gas sensor is non-selectively sensitive to flammable gas components, such as a metal oxide sensor, a Taguchi sensor, an SnO2 sensor, a pellistor sensor, a thermal conductivity sensor, a speed of sound sensor or is a hydrogen selective flammable gas sensor such as an FET sensor.

4. Natural gas and hydrogen detector according to any one of claims 1 - 3, wherein the flammable gas sensor is an FET sensor for selective measurement of low concentrations of hydrogen.

5. Natural gas and hydrogen detector according to any one of the preceding claims, comprising a first flammable gas sensor according to claim 3, and at least a second flammable gas sensor according to claim 3 and different from the first flammable gas sensor.

6. Natural gas and hydrogen detector according to any one of the preceding claims, wherein the flammable gas sensor is not sensitive, less sensitive or less than half as sensitive as the NDIR sensor to any of the gas components to which the NDIR sensor is sensitive.

7. Natural gas and hydrogen detector according to any one of the preceding claims, comprising a first NDIR sensor adapted to detect a first range of hydrocarbon concentrations, and a second NDIR sensor which is different from the first NDIR sensor and adapted to detect a second range of hydrocarbon concentrations being larger than the first range of concentrations.

8. Natural gas and hydrogen detector according to claim 7, wherein the first NDIR sensor comprises a first infrared (IR) sample path adapted to detect hydrocarbon concentrations of above 0.1 volume percent, and wherein the second NDIR sensor comprises a second IR sample path which is longer than the first IR sample path and adapted to detect hydrocarbon concentrations of below 1 volume percent

9. Natural gas and hydrogen detector according to any one of claims 1-6, wherein the NDIR sensor comprises an IR sample path adapted to detect hydrocarbon concentrations in the range of 0 ppm - 100 volume percent, a first optical filter comprising a first passband, such as in the range of 3.3 µm - 3.4 µm, and a second optical filter comprising a second passband different from the first passband, such as in the range of 7.2 µm - 7.4 µm or 3.6 µm - 3.8 µm.

10. Method for detecting the concentration of hydrogen within a gas sample comprising natural gas, such as methane or LPG, and non-hydrocarbon gas components with a natural gas and hydrogen detector according to any of the preceding claims, comprising the following steps:
detecting the amount or concentration of hydrocarbon gas components within the gas sample with the NDIR sensor,
detecting the amount or concentration of flammable gas components, such as hydrogen gas, within the gas sample with the flammable gas sensor,
evaluating the detected amounts or concentrations by comparing the detected hydrocarbon gas amount or concentration with the detected total amount or concentration of flammable gas components, and
considering hydrogen to be present within the gas sample if the amount or concentration of hydrocarbon gas is lower than the total amount or concentration of flammable gas components.

11. Method according to claim 10, comprising
subtracting the hydrocarbon gas amount or concentration from the flammable gas amount or concentration and thereby determining the hydrogen gas amount or concentration, respectively.
